# EUROPEAN PATENT APPLICATION

(11) **EP 1 909 098 A1**
(43) Date of publication of application: **09.04.2008**
(21) Application number: 06767437.4
(22) Date of filing: 27.06.2006
(51) Int. Cl.: G01N 27/327

(54) **BIOSENSOR**

(30) Priority: 27.06.2005 JP 2005185989; 27.06.2005 JP 2005185990; 27.06.2005 JP 2005185991; 14.02.2006 JP 2006035937
(71) Applicant: National Institute of Advanced Industrial Science and Technology, Tokyo 100-8921 (JP)
(72) Inventor: KARUBE, Isao, c/o NATIONAL INST. OF ADVANCED IND, Tsukuba-shi, Ibaraki 305-8562 (JP); GOTOH, Masao, c/o NATIONAL INST. ADVANCED IND, Tsukuba-shi, Ibaraki 305-8562 (JP); NAKAMURA, Hideaki, c/o NATIONAL INST. ADVANCED IND, Tsukuba-shi, Ibaraki 305-8562 (JP)
(74) Representative: Setna, Rohan P.
(86) International application number: PCT/JP2006/312820
(87) International publication number: WO 2007/001003

(57) **Abstract**

There is provided a biosensor (which is integrated with a needle) with a high precision where an assembling is easy and reproducibility of the prepared biosensor (integrated with a needle) is good.

There is provided with a biosensor comprising:
two sheets of electrically insulating substrates connected by a connecting part, and
an electrode and a spacer formed on one of or both of the two sheets of electrically insulating substrates, wherein
the two sheets of the electrically insulating substrates are folded along the connecting part, and
the electrode and the spacer are positioned between the electrically insulating substrates.

In this biosensor (integrated with a needle), a biosensor (integrated with a needle) is able to be easily prepared by folding two sheets of electrically insulating substrates which are connected by a connecting part and, therefore, it achieves excellent effects that the product is able to be easily manufactured without such a troublesome step where each biosensor material is correctly layered and that the biosensor (integrated with a needle) manufactured as such has a good reproducibility and a high precision.

## Description

### Technical Field

The present invention relates to a biosensor (which is integrated with a needle). More particularly, it relates to a biosensor (which is integrated with a needle) by which a component concentration of various kinds of liquid is electrochemically measured utilizing an enzyme, etc. The present invention also relates to a needle integrated biosensor having a constitution in which a puncture needle for piercing the skin for obtaining the blood and a biosensor which collects the body fluid taken out onto the surface of the skin and analyzes it.

### Background Art

With regard to sensor of a disposable type up to now (Patent Document 1 and Patent Document 3), there have been known such ones having a steric structure to ensure the quantitative property and also having a system where capillary phenomenon or the like is utilized (Patent Document 5 and Patent Document 6) whereby a sample solution is automatically introduced into the inner area of a sensor (Patent Document 7). In the sensor of such a constitution, it is assembled by layering of spacer and, further, cover on an electrically insulating substrate. There are installed an electrode pattern on the substrate and an air hole necessary for removal of air required for the capillary phenomenon on the cover. It is necessary that each of those constituting parts is previously punched into a predetermined shape and also that positioning for correct layering of the parts in the steric processing is done whereby, as numbers of the constituting parts increase, steps for the steric processing are complicated. When those sensors further need application of a reagent such as molecule-discriminating element and mediator (Patent Document 2 and Patent Document 4) or formation of a membrane for avoiding the affection by inhibiting substances (Patent Document 8), there is a problem that the steps become more complicated.
Patent Document 1: Japanese Patent Laid-Open No.Sho47-000500
Patent Document 2: Japanese Patent Laid-Open No.Sho48-037187
Patent Document 3: Japanese Patent Laid-Open No. Sho52-142584
Patent Document 4: Japanese Patent Laid-Open No. Sho54-050396
Patent Document 5: Japanese Patent Laid-Open No. Sho56-079242
Patent Document 6: Japanese Patent Laid-Open No. Sho61-502419
Patent Document 7: Japanese Patent Laid-Open No.Hei01-291153
Patent Document 8: Japanese Patent Laid-Open No.Hei03-202764
Patent Document 9: Japanese Patent Laid-Open No.Hei05-199898
Patent Document 10: Japanese Patent Laid-Open No.Hei09-222424
Patent Document 11: Japanese Patent Laid-Open No. 2001-204494
Patent Document 12: WO 01/33216
Patent Document 13: US Patent No. 4,225,410
Patent Document 14: US Patent No. 5,653,864
Patent Document 15: US Patent No. 6,071,391
Non-Patent Document 1 : A. Ahmadian, et al., Biotechniques, 32, 748 (2002)

In the above-mentioned conventional sensors, many steps and materials are needed for the manufacture and there is no other way than to adopt a complicated structure. As a result, much investment for the equipments is needed for the manufacturing lines and yield of the product is not sufficient as well whereby there is much burden in view of the cost. Naturally, environmental loads upon preparation of materials and upon manufacture are big as well. In addition, in terms of characteristics, coefficient of variation (CV) which is a yardstick for imbalance of characteristics of the sensor manufactured is not sufficient as well due to complicated steps and, particularly, positioning upon layering of the substrates. Moreover, since changes in the shape of biosensor cause deterioration of measurement precision and reproducibility, there has been a demand for ensuring the stability in the shape for a long period such as that re-warping, etc. of the cover, etc. are not generated in the biosensor.

In order to solve the above problems, the present inventors formerly proposed a biosensor which is manufactured by a folding process, a bending process or a folding-bending process of a sheet of electrically insulating plane substrate. In this biosensor, electrode is formed on a sheet of electrically insulating substrate and the sheet of plane substrate is processed in a steric manner so that the electrode is aligned in the inner side of the substrate whereby an electrode alignment is made plane or steric and a quantitative measurement in a narrow and small site is made possible. Thus, it is a characteristic feature to constitute the main structure of a sensor from a sheet of plane substrate. However, in such a method, there are problems that attachment of a fixing device to a folded area, thermal adhesion with pressure, cutting, etc. are necessary in order to prevent the warping of the folded area and, since an inlet for introduction of a sample is formed utilizing a dead area of a space formed between the substrate and the cover, a sample liquid is oozed to a groove formed in a boundary of the substrate near the sample introducing inlet and each of the constituting materials for spacer and cover whereby volume of the sample varies.
Patent Document 16: Japanese Patent Laid-Open No. 2005-233917

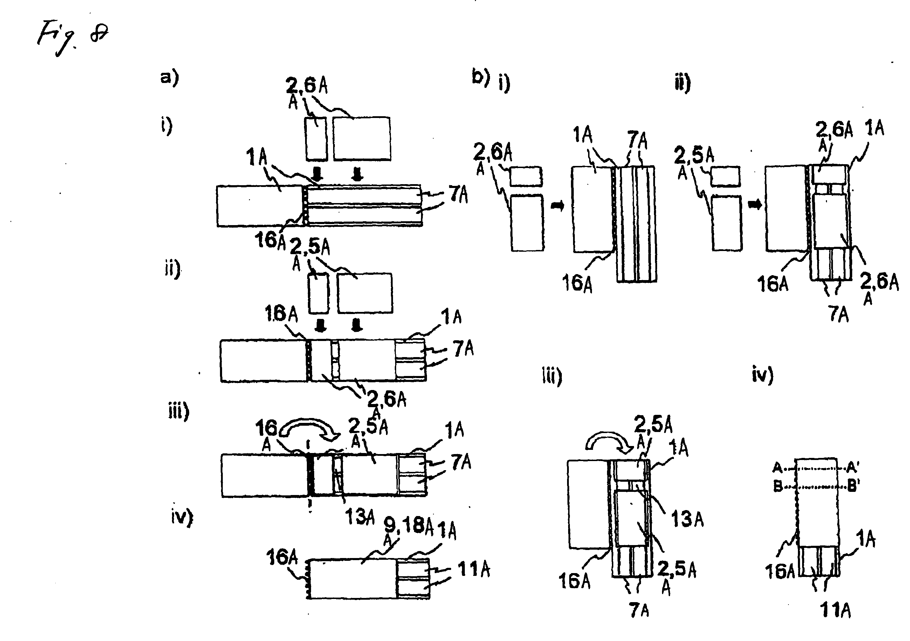

Problems of the above biosensor will be discussed in detail by referring to Fig. 8. Thus, a) and b) show examples for assembling the conventional biosensor in which only the shape of the substrate 1A is different. In i), there is shown a sheet of substrate 1A where electroconductive materials 7A, 7A are formed on the surface and a perforation 16A which is a folding part is installed and also a resist layer 6A coated thereby. The resist layer 6A also acts as a spacer 2A. In ii), there is shown a substrate 1A where a resist layer is formed on the surface and an adhesive layer 5A which is coated in the next assembling step. Here, the adhesive layer 5A also acts as a spacer 2A as same as the resist layer 6A. In iii), there is shown the state before the substrate where an adhesive layer 5A is formed on the surface is folded along the perforation 16A and is layered. In iv), there is shown a biosensor 3A which is a folded molding 14A formed by the substrate 1A. In that case, the folded area formed along the perforation 16A may be warped by the thickness of the spacer such as the resist layer 6A or the adhesive layer 5A and, therefore, it is necessary to conduct some treatment such as to attach a fixing device to this area or to remove the re-warping stress by means of thermal adhesion with pressure.

As mentioned above, in the sensors of a folding type as such, although it is successful in greatly improving the conventional method for the manufacture of sensors by much simplification of the manufacturing steps, elimination of materials, adoption of very simple structure, etc., the sensor formed by the manufacturing method needs attachment of fixing device to the folded area, thermal adhesion with pressure, cutting, etc. in order to prevent the warping of the folded area and there has been a demand for the improvement.

Up to now, there have been cases where a patient himself/herself suffering from diabetes mellitus collects his/her blood and measures the blood sugar level which is a glucose amount in the blood. In that case, the patient uses a blood collecting instrument called a lancet where a blood collecting needle is attached and detached to collect the blood by piercing his/her finger or arm and transfers the collected blood to a blood sugar level analyzer to measure the blood sugar level. According to such a measuring system, the patient must carry a set of measuring instrument comprising several items such as blood sugar level analyzer, lancet, blood collecting needle and analyzing element and conduct the measurement by assembling them upon necessity. Thus, a long period of training is needed for its operation and considerable time is required until a reliable measurement is able to be conducted by the patient himself/herself. In fact, measurement at the site other than fingers and forearms (such as abdominal wall and earlobe) is difficult for even skilful persons. In recent years, due to the needs for providing a minimally invasive sample having less pain, a biosensor by which measurement is possible where the amount of the sample is 1 µl or less has been developed and, in the case of such a very small amount, a work for a correct supply of a sample to a biosensor is very difficult. As a result, an error in the measurement is resulted and there are inconveniences that the patient who is a person to be measured must be punctured once again and that a biosensor must be also exchanged to make a fresh start for the measurement.
Patent Document 17: Japanese Patent No. 3,621,502
Patent Document 18: Examined Japanese Patent Application Publication No.Hei08-020412

Under such circumstances, various needle integrated biosensors have been devised. Firstly, in a needle integrated biosensor mentioned in the Patent Document 19, each of a puncture needle and a biosensor is set at different positions in an inner area of a measuring apparatus of a pen type (like a two-colored ball point pen) equipped with a driving part for a puncture needle and, after the front end of the pen-shaped measuring apparatus is applied to the skin of a person to be tested and pierced, a biosensor is exposed at the front end to collect the blood whereupon measurement of blood sugar level is conducted. In this method however, troublesomeness that each of the needle and the biosensor must be set onto a measuring apparatus has been still unsolved.
Patent Document 19: Japanese Patent Laid-Open No. 2000-217804

In another needle integrated biosensor as mentioned in the Patent Document 20, a puncture needle is subjected to driving from outside and there is adopted an integrated structure where a puncture needle moves parallel along a longitudinal direction of a slender biosensor in a small piece. However, in such a type, since substrate, cover, etc. are to be layered to form a needle integrated biosensor, there are many manufacturing steps such as a step where the positions to be layered are to be correctly adjusted. Since it is difficult to correctly layer each of the biosensor materials, difference is noted in reproducibility among different lots and there is a problem that it is difficult to prepare biosensors in high precision.
Patent Document 20: Japanese Patent Re-Laid-Open No. 2002-056769

Up to now, there have been cases where a patient himself/herself suffering from diabetes mellitus collects his/her blood and measures the blood sugar level which is a glucose amount in blood. In that case, the patient uses a blood collecting instrument called a lancet where a blood collecting needle is attached and detached to collect the blood by piercing his/her finger or arm and transfers the collected blood to a blood sugar level analyzer to measure the blood sugar level. According to such a measuring system, the patient must carry a set of measuring instrument comprising several items such as blood sugar level analyzer, lancet, blood collecting needle and analyzing element and conduct the measurement by assembling them upon necessity. Thus, a long period of training is needed for its operation and considerable time is required until a reliable measurement is able to be conducted by the patient himself/herself. In fact, measurement at the site other than fingers and forearms (such as abdominal wall and earlobe) is difficult for even skilful persons. In recent years, due to the needs for providing a minimally invasive sample having less pain, a biosensor by which measurement is possible where the amount of the sample is 1 µl or less has been developed and, in the case of such a very small amount, a work for a correct supply of a sample to a biosensor is very difficult. As a result, a error in the measurement is resulted and there are inconveniences that the patient who is a person to be measured must be punctured once again and that a biosensor must be also exchanged to make a fresh start for the measurement.
Patent Document 21: Japanese Patent Laid-Open No.Hei09-266898
Patent Document 22: Examined Japanese Patent Application Publication No.Hei08-020412

Under such circumstances, various needle integrated biosensors have been devised. Firstly, in a needle integrated biosensor mentioned in the Patent Document 23, each of a puncture needle and a biosensor is set at different positions in an inner area of a measuring apparatus of a pen type (like a two-colored ball point pen) equipped with a driving part for a puncture needle and, after the front end of the pen-shaped measuring apparatus is applied to the skin of a person to be tested and pierced, a biosensor is exposed at the front end to collect the blood whereupon measurement of blood sugar level is conducted. In this method however, troublesomeness that each of the needle and the biosensor must be set onto a measuring apparatus has not been solved.
Patent Document 23: Japanese Patent Laid-Open No. 2000-217804

In another needle integrated biosensor as mentioned in the Patent Document 24, a puncture needle is subjected to driving from outside and there is adopted an integrated structure where a puncture needle moves parallel along a longitudinal direction of a slender biosensor in a small piece. However, in such a type, since the collected blood is sent to the whole space occupying both of the sample-conveying path and the puncture-needle path, collection of the blood in more than necessary amount is needed. Further, since the whole shape of the needle integrated biosensor is bilaterally symmetric, there is a risk that the user makes a mistake in its insertion into a measuring apparatus equipped with a puncture driving.
Patent Document 24: Japanese Patent Re-Laid-Open No. 2002-056769

As such, in the conventional needle integrated biosensor, an electrode system is formed on the plane substrate and, therefore, the structure is plane and, due to the necessity of filling this plane with a sample liquid, there is a problem that the sample volume becomes too much as a result thereof.

### Disclosure of the Invention

### Problems to be solved by the Invention

An object of the present invention is to provide a biosensor (which is integrated with a needle) with a high precision where an assembling is easy and reproducibility of the prepared biosensor (integrated with a needle) is good. Another object of the present invention is to provide a needle integrated biosensor where suppression of collecting amount of the blood after piercing is made possible and there is no risk that users will make a mistake for its insertion into a measuring apparatus. Still another object of the present invention is to provide a needle integrated biosensor where the collecting amount of blood after piercing is able to be made a minimum necessary one.

### Means for solving the Problems

The object of the present invention is achieved by a biosensor including:
two sheets of electrically insulating substrates connected by a connecting part,
an electrode and a spacer formed on one of or both of the two sheets of electrically insulating substrates, wherein
the two sheets of the electrically insulating substrates are folded along the connecting part, and
the electrode and the spacer (and a puncture needle for collection of a body fluid by piercing a skin of a person to be tested) are positioned between the electrically insulating substrates.

Further, the object of the present invention is achieved by a needle integrated biosensor including:
a biosensor including an electrode and a spacer in a space sandwiched between two sheets of electrically insulating substrates, and
a puncture needle which is provided in the biosensor to collect a body fluid by piercing a skin of a person to be tested,
the biosensor and the puncture needle being constituted in an integrated manner via a support for the puncture needle, wherein
the puncture needle is provided in a crossing manner at a right angle to the long-axial direction of the electrode formed on one of the substrates and, preferably, the biosensor has a bilaterally asymmetric shape where the puncture needle is a center line.

Further, the object of the present invention is achieved by a needle integrated biosensor including:
a biosensor including an electrode and a spacer in a space sandwiched between two sheets of electrically insulating substrates, and
a puncture needle which is provided in the biosensor to collect a body fluid by piercing a skin of a person to be tested,
the biosensor and the puncture needle being constituted in an integrated manner via a support for the puncture needle, wherein
the electrodes are provided in a face-to-face manner to form facing electrodes.

### Effects of the Invention

In the biosensor (integrated with a needle) in accordance with the present invention, since a biosensor (integrated a needle) is able to be easily prepared by folding two electrically insulating substrates connected by a connecting part, there are achieved excellent effects that its manufacture is easy without a troublesome step where each biosensor material is correctly folded and that, at the same time, the prepared biosensor (integrated with a needle) has an excellent reproducibility and a high precision. When a pole insertion hole is formed through which a pole used for positioning upon formation of a biosensor is penetrated on the two insulating substrates, molding by means of folding is able to be conducted more precisely. The biosensor (integrated with a needle) which is able to be easily assembled as such has also an effect that a large-scale production is possible.

Further, since a puncture needle is aligned in a manner of crossing at right angles to the long-axial direction of the electrode in the needle integrated biosensor in accordance with the present invention, there is achieved an excellent effect that an efficient measurement is possible without collection of the blood in an amount of more than necessary one after piercing. When it is made into a bilaterally asymmetric shape where a puncture needle is a center line, an erroneous insertion into a measuring apparatus in the actual use is able to be prevented.

Still further, since the electrodes have a facing structure where the electrodes are in a face-to-face manner in the needle integrated biosensor in accordance with the present invention, there is achieved an excellent effect that an efficient measurement is possible without collection of the blood in an amount of more than necessary one after piercing. When it is made into a bilaterally asymmetric shape where a puncture needle is a center line, an erroneous insertion into a measuring apparatus in the actual use is able to be prevented.

### Brief Description of the Drawings

Fig. 1 is a drawing which shows an example for assembling the folded biosensor according to the present invention.
Fig. 2 is a drawing which shows an example for molding the folded biosensor according to the present invention.
Fig. 3 is a drawing which shows an example for molding the folded biosensor according to the present invention.
Fig. 4 is a drawing which shows an example for the manufacture of the folded needle integrated biosensor according to the present invention.
Fig. 5 is a drawing which shows an example for the constitution of the folded needle integrated biosensor according to the present invention.
Fig. 6 is a drawing which shows an example for the use of the folded needle integrated biosensor according to the present invention.
Fig. 7 is a drawing which shows another example for the manufacture of the folded needle integrated biosensor according to the present invention.
Fig. 8 is a drawing which shows an example for assembling the conventional biosensor.
Fig. 9 is a drawing which shows an example for assembling the needle integrated biosensor according to the present invention.
Fig. 10 is a drawing which shows an example for constituting the needle integrated biosensor according to the present invention.
Fig. 11 is a drawing which shows an example for using the needle integrated biosensor according to the present invention.
Fig. 12 is a drawing which shows another example for assembling the needle integrated biosensor according to the present invention.
Fig. 13 is a drawing which shows another example for constituting the needle integrated biosensor according to the present invention.
Fig. 14 is a drawing which shows another example for using the needle integrated biosensor according to the present invention.
Fig. 15 is a drawing which shows an example for assembling the needle integrated biosensor according to the present invention.
Fig. 16 is a drawing which shows an example for constituting the needle integrated biosensor according to the present invention.
Fig. 17 is a drawing which shows an example for using the needle integrated biosensor according to the present invention.
Fig. 18 is a drawing which shows another example for assembling the needle integrated biosensor according to the present invention.
Fig. 19 is a drawing which shows another example for constituting the needle integrated biosensor according to the present invention.
Fig. 20 is a drawing which shows another example for using the needle integrated biosensor according to the present invention.

### Explanation of referential numerals

- 1A: substrate
- 2A: spacer
- 3A: needle integrated biosensor
- 4A: penetration hole
- 5A: adhesive layer
- 6A: resist layer
- 7A: electrically conductive material
- 8A: sample-conveying path (puncture-needle path)
- 10A: electrode
- 11A: terminal
- 12A: opening for piercing and collecting blood
- 13A: electrode reactive part
- 14A: puncture needle part
- 16A: perforation
- 17A: connecting part to outer drive

- 18A: folded molding
- 19A: support for a puncture needle
- 20A: puncture needle
- 21A: connecting part
- 24A: collection of the blood
- 25A: folding guide
- 26A: pole
- 27A: hinge
- 28A: receiving part for a pole
- 29A: insertion hole for a pole
- 30A: connecting part
- 31A: elastic substance
- 32A: supporting plate
- 1B: lower substrate
- 2B: spacer
- 3B: needle integrated biosensor
- 4B: penetration hole
- 5B: adhesive layer
- 6B: resist layer
- 7B: electrically conductive material
- 8B: sample-conveying path/puncture-needle path
- 10B: electrode
- 11B: terminal
- 12B: opening for piercing and collecting blood
- 13B: electrode reaction part (reagent layer)
- 14B: puncture needle part
- 15B: soft material
- 16B: shrinkable material
- 17B: connecting part to outer drive
- 18B: folded molding
- 19B: support for a puncture needle
- 20B: puncture needle
- 21B: connecting part
- 22B: hook
- 23B: adhesive part
- 24B: collection of the blood
- 1C: substrate
- 2C: spacer
- 3C: needle integrated biosensor
- 4C: penetration hole
- 5C: adhesive layer
- 6C: resist layer
- 7C: electrically conductive material
- 8C: sample-conveying path/puncture-needle path
- 10C: electrode
- 11C: terminal
- 12C: opening for piercing and collecting blood
- 13C: electrode reaction part (reagent layer)
- 14C: puncture needle part
- 15C: soft material
- 16C: shrinkable material
- 17C: connecting part to outer drive
- 18C: folding molded material
- 19C: support for a puncture needle
- 20C: puncture needle
- 21C: connecting part
- 22C: hook
- 23C: adhesive part
- 24C: collection of the blood

### Best Mode for Carrying Out the Invention

As to a substrate, an electrically insulating one is sufficient and, for example, plastic, biodegradable material, paper, etc. is used therefor. Preferably, polyethylene terephthalate is used therefor where a product in which two sheets of substrates are connected by a connecting part is used. As to a connecting part, there are used that where its length is not less than the thickness of a spacer which will be mentioned later, i.e. 0.5 to 5 mm and its width is 0.2 to 2.5 mm or, preferably, its length is 1.0 to 4 mm and its width is 0.5 to 1.5 mm and, in the case of a needle integrated biosensors which is a biosensor where a puncture needle is integrated, that where its length is 1 to 6 mm and its width is 0.2 to 3 mm or, preferably, its length is 1.5 to 5 mm and its width is 0.5 to 2 mm is installed at least in two places preferably between two sheets of substrates. When the length is about 0.5 to 0.9 mm, such a connecting part is formed as a broken line on the insulating substrate using, for example, a cogwheel-shaped thin disk where its convex part is a blade while, in the case of a connecting part with a length of about 1 to 6 mm, the electrically insulating substrate is punched with a mold to subject to a hinge molding. Accordingly, in the present invention, the two sheets of electrically insulating substrates mean each of the substrates where a connecting part is formed on one sheet of insulating substrate whereby a substrate is formed in which the connecting part is a boundary. In this case, when length of the connecting part is made not shorter than 0.5 mm, the necessity for fixing the folded part by means of thermal adhesion with pressure or using a fixing device lowers and, particularly in the case of a needle integrated biosensor where length is 1 to 4 mm and width is about 0.5 to 1.5 mm, it is no longer necessary to prevent the warping of the folded part by means of thermal adhesion with pressure or using a fixing device in case the length and width of the connecting part are made about 1.5 to 5 mm and 0.5 to 2 mm, respectively. There are some cases where the precision upon folding becomes a bit deteriorated when the length of the connecting part is made longer but, in those cases, such an inconvenience is able to be avoided by conducting the positioning of the two insulating substrates as will be mentioned below.

On two sheets of insulating substrates, pole penetrating holes through which a pole preferably used for positioning of the two substrates penetrates are installed at the facing sites in the formation of the biosensor. As a result of formation of such a penetrating hole, there are achieved excellent effects that it is now possible to easily carry out the positioning of the two substrates by penetration of the pole into the pole-inserting hole in the formation of the biosensor and that, at the same time, it is now possible to provide a biosensor (integrated with a needle) with a high precision having an excellent reproducibility of the prepared biosensor.

The electrode is formed on a substrate by a screen printing method, a vapor deposition method, a sputtering method, a foil adhesion method, a galvanizing method, etc. and, as to a material therefor, carbon, silver, silver/silver chloride, platinum, gold, nickel, copper, palladium, titanium, iridium, lead, tin oxide and platinum black may be exemplified. As to the carbon hereinabove, carbon nanotube, carbon microcoil, carbon nanohone, fullerene, dendrimer or a derivative thereof may be used.

The electrode may be a two-electrode method formed by working electrode and counter electrode, a three-electrode method formed by working electrode, counter electrode and reference electrode or an electrode method formed by more electrode numbers. When a three-electrode method is adopted, a transfer speed of the collected blood introduced into a conveying path is able to be measured in addition to an electrochemical measurement of the substance to be measured whereby a hematocrit value is able to be measured. It is also possible to constitute from two or more sets of electrode system.

Electrodes are in a facing structure being aligned in a face-to-face manner or, to be more specific, in a facing structure that electrodes formed on two substrate surfaces sandwich a spacer comprising a resist layer, an adhesive layer, etc. Due to the above, electrochemical reaction efficiently proceeds and volume of the reaction layer is able to be effectively made small by shortening of the distance between electrodes and shortening of the electrode area and, as a result, amount of the sample is able to be made small.

Although those electrodes are formed together on a sheet of substrate or separately on two sheets of substrate, a facing structure where electrode is oppositely aligned on two substrates or, to be more specific, a facing structure where electrodes formed on the surfaces of two substrates sandwich a spacer comprising a resist layer or an adhesive layer is preferred from the viewpoint of making the sample volume small. As a result thereof, electrochemical reaction proceeds efficiently and volume of the reaction layer can be effectively made small by shortening of distance between electrodes and of electrode area whereby it is possible to make the amount of the sample small.

A reagent layer (electrode reaction part) is formed on a substrate where electrode is formed. The reagent layer is formed by a screen printing method or a dispenser method and fixation of the reagent layer onto the electrode surface or the substrate surface is able to be conducted by a covalent bond method or an adsorbing method accompanied by drying. With regard to a reagent aligned to the electrode reaction part of the biosensor, that which contains glucose oxidase which is an oxidizing enzyme and potassium ferricyanide as a mediator may be exemplified when constitution is done for the measurement of blood sugar level. When the reagent is dissolved in blood, an enzymatic reaction starts and, as a result, potassium ferricyanide coexisting in the reaction layer is reduced and potassium ferrocyanide which is an electron transmitter of a reduced type is accumulated. Its amount is proportional to the substrate concentration or, in other words, the concentration of glucose in the blood. The electron transmitter of a reduced type which is accumulated for a predetermined period is oxidized by electrochemical reaction. An electron circuit in the main body of the measuring apparatus which will be mentioned later calculates and determines the glucose concentration (blood sugar level) from the positive electrode current measured at that time and displays it onto the display aligned on the surface of the main body.

Surfactant and lipid may also be applied around the opening for collecting the blood and on the surface of electrode or reagent layer (electrode reaction part). As a result of application of the surfactant and the lipid, it is now possible to make the transfer of the sample smooth.

Here, in the needle integrated biosensor, as a result of application of reagent layer, surfactant or lipid into the sample-conveying path, there is a possibility that a puncture needle contained therein is contaminated. In order to prevent the pollution as such, it is preferred that such a reagent is not applied around the front end of a puncture needle.

In the biosensor where a reagent layer is equipped on the electrode where the above blood collection is satisfied, the collected blood reacts with the reagent when the collected blood sent from the opening for collecting the blood contacts to the reagent layer on the electrode. This reaction is monitored as electric changes in the electrode.

It is also possible that the electrode of the biosensor is provided by a resist layer and the resist layer is also able to be easily formed by a screen printing or the like. There is no particular limitation for the resist in that case provided that it does not react with or is not dissolved in the substrate and its examples are an ultraviolet-hardening vinyl-acrylic resin, a urethane-acrylic resin and a polyester-acrylic resin. Main object for the use of a resist is to make the electrode pattern clear and to clarify the stipulation of the above electrode area and another object is to insulate the sample-conveying path where no reagent layer is present. For that purpose, the resist layer may or may not form the same pattern as the adhesive layer. In the latter case, it is preferred that the resist layer is formed on an electrode substrate for the sake of insulation. Further, when the resist layer is formed thicker than the electrode in a sample-conveying path where the puncture needle of the needle integrated biosensor according to the present invention is received, contact of the puncture needle to the electrode is able to be suppressed. Such a resist layer is also able to be formed by a screen printing method and, for example, a resist layer formed by any of the above materials in the thickness of about 50 to 500 µm or, preferably, about 10 to 100 µm also acts as a spacer.

Since the two substrates connected by the connecting part are adhered by an adhesive, an adhesive layer is formed on one of or both of the two insulating substrates. The adhesive layer may be acceptable provided that it does not react with or dissolve in the cover as same as in the case of the resist layer and there is no particular limitation therefor where its example is an adhesive of an acrylate resin type. Such an adhesive layer is also able to be formed by a screen printing method and the adhesive layer formed in the thickness of about 5 to 500 µm or, preferably, about 10 to 100 µm also acts as a spacer. Incidentally, it is also possible that the above reagent is contained in the adhesive layer.

When the substrate having the above constitution in which a puncture needle is installed is folded along a connecting part, a biosensor as a folded molding product is able to be manufactured. As to the connecting part, that having a length of not shorter than the thickness of a spacer or, 0.5 to 4 mm and, preferably, 1.0 to 3. 0 mm is placed preferably between the two substrates in at least two places. The connecting part as such in a length of about 0.5 to 0.9 mm is able to be formed as a perforation in a broken line on an insulating substrate using, for example, a cogwheel-shaped thin disk where its convex part is a blade while, in the case of a connecting part in a length of about 1 to 4 mm, the insulating substrate is punched with a mold to subject to a hinge molding. When the connecting part is in a length of about 1 to 4 mm, it is not necessary that the folded area is subjected to a thermal adhesion with pressure or fixed using a fixing device so as to prevent a backward bending. In the case of a biosensor which is a folded molding product as such, it is possible to manufacture a lot of biosensors at a time by such a means that a connecting part as a folding line is formed in a horizontal way to a direction of long axis of a long substrate, then electrode, etc. are formed, folding along the connecting part is conducted and punching into a shape of a sensor is done. In a needle integrated biosensor which is manufactured by such a method, reproducibility is also very good and it has advantages which have not been achieved in the conventional layered method.

A biosensor as a folded molding is manufactured when the two electrically insulating substrates where electrode and spacer are formed are folded along the connecting part or, preferably, when the folding along the connecting part is conducted together with penetration of a pole for positioning into a pole insertion hole installed on the insulating substrate. In the case of a biosensor which is a folded molding as such, it is now possible to manufacture many biosensors at a time when a connecting part which is a folding line is formed being horizontal in the long-axial direction of a long substrate, electrode, etc. are then formed, folding is done along the connecting part and then punching into a shape of a sensor is done. In the biosensor prepared by such a manufacturing method, reproducibility is very good and it has a characteristic which has not been available in the conventional layering method.

In the biosensor having the above constitution, it is also possible to prepare a needle integrated biosensor where a puncture needle for collecting the body fluid by piercing the skin of a person to be tested upon folding the insulating substrate is also included together with electrode and spacer whereby electrode, spacer and puncture needle are positioned between the insulating substrates.

In a biosensor, a puncture needle for piercing the skin of the person to be tested and to collect the body fluid is installed. Although the puncture needle may be in any alignment such as in parallel or at right angles to the electrode, it is preferably placed in a state of crossing the electrode at the angles to the electrode. When a puncture needle is placed in a state of being right angles to the electrode, amount of the collecting blood is able to be suppressed as compared with the case where the puncture needle is placed in parallel to the long axial direction of the electrode. When the puncture needle is placed at right angles to the electrode, the terminal for measurement is placed at the position which is apart from the orbit of the puncture needle whereby it is possible to make the shape of a needle integrated biosensor bilaterally asymmetric along the puncture needle as a central line. Accordingly, a user is able to adopt it as a mark so that an error for right and left in inserting into a measuring apparatus is able to be avoided and the measuring apparatus is also able to be equipped with a mechanism for specifying the position of the terminal of the needle integrated biosensor according to the present invention.

During the manufacture of a biosensor having the above-mentioned constitution, it is constituted in such a manner that, under a condition of negative pressure than the outer air or, preferably, under a vacuum condition, an opening for piercing and collecting blood is collected by piercing is covered by a soft material such as silicone rubber, soft polyurethane, polyvinyl chloride or foamed polystyrene while a space between a support for a puncture needle and two substrates constituting the biosensor at the side of a piercing drive is constituted so as to give a tightly closed state by a shrinkable material such as natural rubber whereby the inner area of the sensor is tightly closed in a negative pressure state and, in the transfer of the collected blood to a sample-conveying path after piercing, a suction means is able to be used in addition to a capillary phenomenon. At that time, when a puncture needle is further pulled in the direction opposite to the piercing direction immediately after the piercing, the shrinkable material is extended and the collected blood is able to be sucked under the state where the inner negative pressure is further enhanced. As a result of adoption of the constitution as such, it is now possible to smoothly conduct the collection of the blood. Here, in the needle integrated biosensor according to the present invention, since the terminal is able to be made outside the orbit of the puncture needle as mentioned above, it is easy to give a structure for keeping the airtightness so that the inner area of the sample-conveying path containing a puncture needle is kept in negative pressure. The soft material covering the opening for piercing and collecting the blood also achieves an effect of improving the close adhesion of the skin of the person to be tested with the opening for piercing and collecting the blood in addition to the effect of maintaining the negative pressure.

With regard to the puncture needle, since it is necessary to pierce the person to be tested, it is desired to have the strength durable for that and to be sharp and, in order to suppress the pain upon piercing, it is desired to be a fine piercing needle. To be more specific, a product of Terumo of 21 to 33 gauges is used. The puncture needle may be either hollow or rod-shaped so far as it is able to pass through the skin of a person to be tested. Since the puncture needle is to be hygienically received in a biosensor until its use, a photocatalytic function having antibiotic and antiviral effects may be applied to the surface of front end of the needle. In that case, titanium oxide or a film of titanium oxide is preferred.

In the needle integrated biosensor as such, it is desirable that the biosensor is set in a measuring apparatus equipped with a piercing drive whereby a series of operations of piercing, blood collection and measurement is carried out. In that case, it is desirable that the piercing drive, for example, is equipped with a mechanism where a needle breaks through the skin of the person to be tested and a mechanism where it quickly returns to the original position immediately after the piercing.

When the needle integrated biosensor according to the present invention is equipped with a sucking mechanism, a piercing drive system in the measuring apparatus may be further improved for enhancing the sucking force upon collection of the blood. Thus, a puncture needle may be further pulled in the direction which is opposite to the piercing direction using a mechanism in the direction of returning the alignment of the puncture needle immediately after the piercing so that a shrinkable material is extended and the negative pressure inside is made higher.

With regard to a measuring apparatus for a needle integrated biosensor, there is used an apparatus in which operability and durability for surely and repeatedly conducting the measurement using the needle integrated biosensor are ensured and which is able to be easily carried and there is also used a measuring apparatus having such a function that a state where the measurement is possible is resulted when a needle integrated biosensor is inserted into an introducing part on the lower area whereby the support for a puncture needle turns upside and the terminal of the biosensor is connected to a connector of the measuring apparatus, then preparation of the measurement is completed when a trigger is pulled so as to give a piercing drive to the inner part of the needle integrated biosensor, then piercing, blood collection and measurement are automatically done in this order by pushing the switch of the piercing initiation button and the measuring result is induced finally.

An example of the characteristics of the measuring apparatus will be mentioned in more detail. In this measuring apparatus, a driving part for a puncture needle and a measuring apparatus part are integrated and the driving part for a puncture needle is constituted from a trigger part, a piercing initiation button part and a driving part made of elastic substances such as spring. On the other hand, in the measuring apparatus part, its fundamental constitution comprises a sensor introducing part, a connector, a circuit for electrochemical measurement, a memory part, an operation panel, a measuring part which measures electric value of the biosensor at the electrode and a display part which displays the measured value at the measuring part and is further able to be installed with electric wave as a wireless means such as Blue Tooth (registered trade mark). Due to such a slide structure, a piercing drive is applied keeping the state where the needle integrated biosensor is surely held whereby the strength of the measuring apparatus as a whole is able to be enhanced. The measuring apparatus is also able to be equipped with a mechanism which is able to recognize the asymmetric structure where a puncture needle of a needle integrated biosensor is a central line at the projected part of the terminal for the measurement.

With regard to a piercing drive of the measuring apparatus, a mechanism where, after the upper part of the needle integrated biosensor is struck in the vertical direction, it quickly returns and it is further preferred that the drive has a mechanism where depth of piercing the skin of the persons to be tested is able to be adjusted.

The measuring apparatus is also able to be equipped with a voice guidance function and a voice recognizing function corresponding to the visual impairment caused by diabetes mellitus, a control function for measured data by incorporation of a radio clock, a communication function of measured data, etc. to medical organizations, etc., a charging function and others.

Although there is no particular limitation for the measuring method at the measuring part of the measuring apparatus, there may be used potential step chronoamperometry, coulombmetry, cyclic voltammetry, etc.

In view of the above, the needle integrated biosensor in accordance with the present invention is able to cope with a universal plan where the users are not restricted.

Now each of the needle integrated biosensors in the embodiments according to the present invention will be illustrated in detail by referring to drawings and the present invention is not limited to the following examples so far as they are within a gist of the present invention.

### Example 1

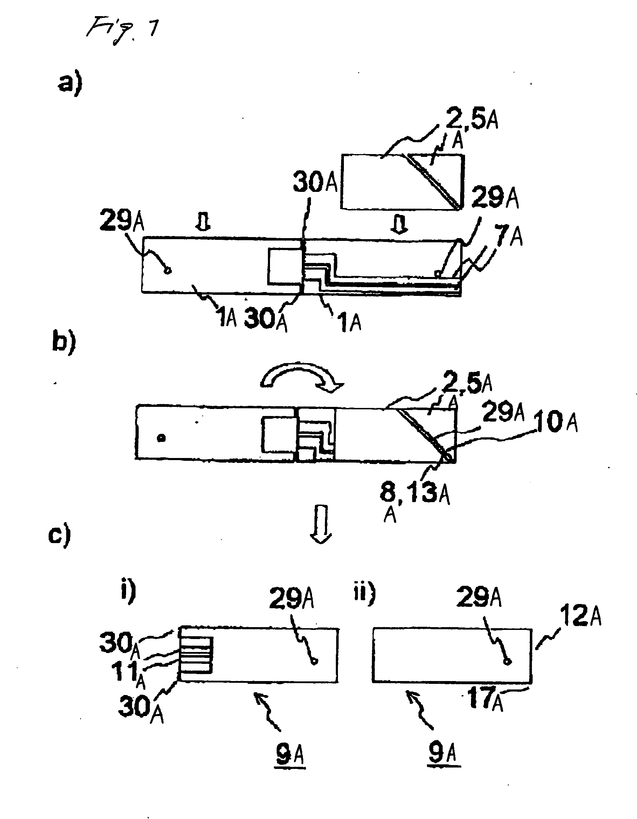

Fig. 1 shows an example for assembling the biosensor according to the present invention. In a), there are shown electrically insulating substrates 1A, 1A where a connecting part 30A is formed between two electrically insulating substrates and there is also shown an adhesive layer 5A. Here, in each of the two substrates, a pole inserting hole 29A functioning as a position hole upon folding molding is formed. When lengths of the two substrates are same, there may be the cases where strain is resulted due to the thickness of the spacer, etc. whereby the terminals of the substrates do not fit together and, at that time, the terminals when both are adhered are now able to be made identical by making one of the substrates a bit longer. A space between the two adhesive layers 5A is a part where an electrically conductive material 7A formed on the substrate 1A becomes an electrode and thickness of the adhesive layer forms a spacer 4A. Accordingly, with regard to the adhesive layer 5A shown here, its thickness plays an important role for stipulating the volume of the sample. In b), there is shown a manner where the adhesive layer 5A does not clog the pole insertion hole 29A formed on the electrically insulating substrate 1A. In the case of the biosensor 9A as such, the area wherefrom the blood is collected 17A is made easy to understand by forming an opening 17A for collecting blood at the corner of the sensor. Further, the above pole insertion hole 29A is installed in more inner area than the electrode 10A when seen from the opening for collecting blood in the sample-conveying path 8A and, therefore, it has an effect that it also acts as an discharging outlet for air 14A for preventing the introduction of more than necessary amount of the collected blood. When the electrically insulating substrates 1A, 1A are folded according to an arrow shown in b) so that the pole insertion holes formed on the two substrates fit, a biosensor 9A as shown in c) is formed. In c)i), there is shown a front view of the biosensor 9A while, in ii), there is shown a back view thereof. Since one of the substrate ends is concave, a state where an electroconductive material formed on another substrate is exposed is resulted and that forms a terminal 11A connecting to the measuring apparatus. Depending upon the pattern of the formed electrode, it is also possible to use a convex insulating substrate instead of a concave one.

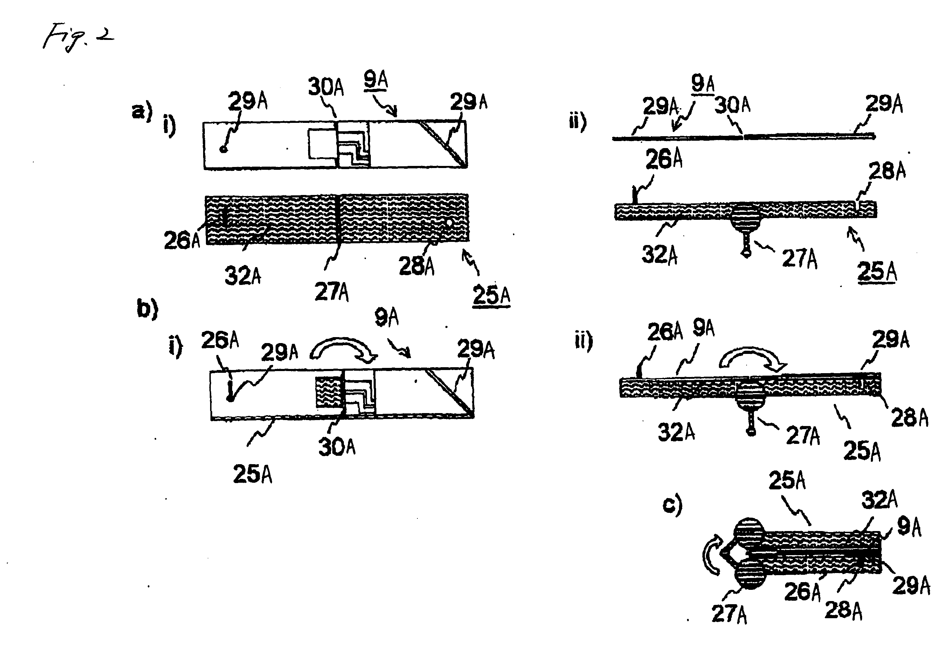

Fig. 2 shows an example of molding of the folded biosensor shown in Fig. 1. Fig. 2a) shows a folding guide 25A which guides the folding molding of the biosensor before the folding molding as shown in Fig. 1 and also of the biosensor 9A. Fig. 2a)i) shows front views thereof and Fig. 2a)ii) shows side views thereof. In the folding guide 25A, there are a pole 26A for inserting into a pole insertion hole 29A of the biosensor 9A, a pole receiving part 28A for receiving the pole 26A and a guide plate 32A which is able to be opened and closed by means of a hinge 27A. Fig. 2b) shows the state where a needle integrated biosensor 9A before the folding molding is set on the folding guide 25A in such a manner that a pole is inserted into one of the pole insertion holes 29A formed on the substrate and another pole insertion hole 29A fits the pole receiving part 28A formed on the folding guide 25A. Fig. 2c) shows a state where the folding guide 15A is folded and layered under the above state. Here, a pole 26A on the folding guide 25A is correctly received in a pole receiving part 28A whereby there is achieved an effect that a folding molding of a biosensor is able to be carried out more precisely.

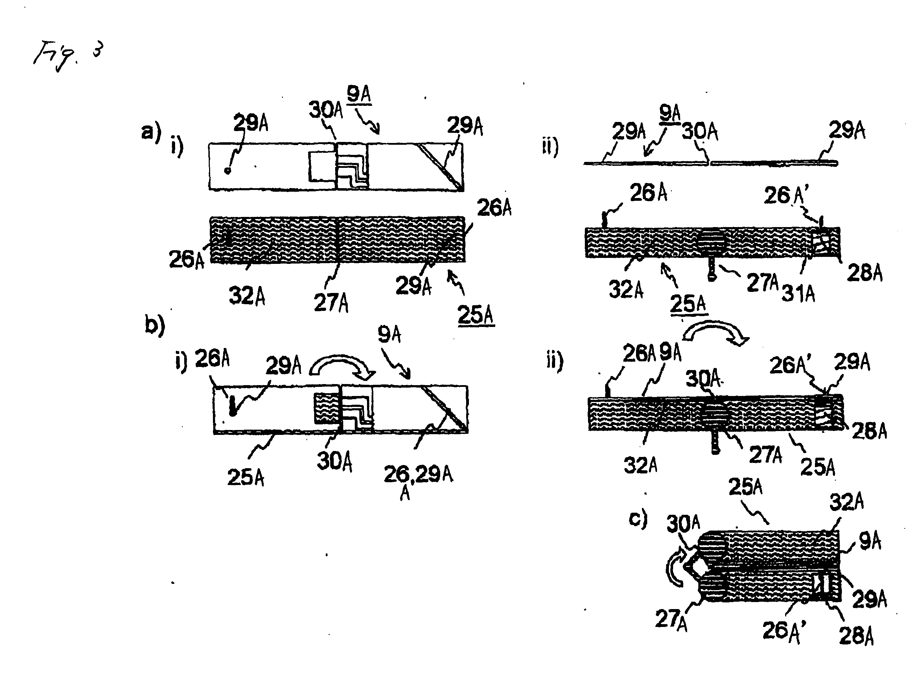

Fig. 3 shows another example of a molding method of the folded biosensor shown in Fig. 1. The difference from Fig. 2 is that, in order to surely receive the pole 26A in a pole receiving part 28A installed in the guide 25A, a pole 26A' is also installed on the upper part of the pole receiving part 28A. The front end of this pole 26A is concave whereby the connection to a round front end of another pole 26A is made sure. Further, in the pole receiving part 28A of the lower area of the concave pole 26A' , an elastic material such as coiled spring is aligned and there is such a structure that, when the two folding guides 25A are folded and piled, weight is applied to the concave pole 26A' after the convex pole 16A is connected to the concave pole 26A' whereby the spring shrinks. As a result, the convex pole 26A is in such a structure that it is received in the pole receiving part 28A under the state of being connected to the concave pole 26A'. According to the manufacturing method shown in this drawing, the substrates are able to be doubled in a sure arrangement under that state where the upper and lower substrates of the biosensor are fixed by poles 26A, 26A' aligned to the folding guide 25A and, therefore, although the mechanism is a bit complex as compared with Fig. 2, it is able to be said to be a method having a very high precision in adhesion. In this method, it is also possible to further make the folding precision better by installing magnets or electrode stones having different polarities at the front ends of the convex pole 26A and the concave pole 26A' or by installing electric terminals so that the connecting condition of both is judged by the degree of continuity due to connection to both.

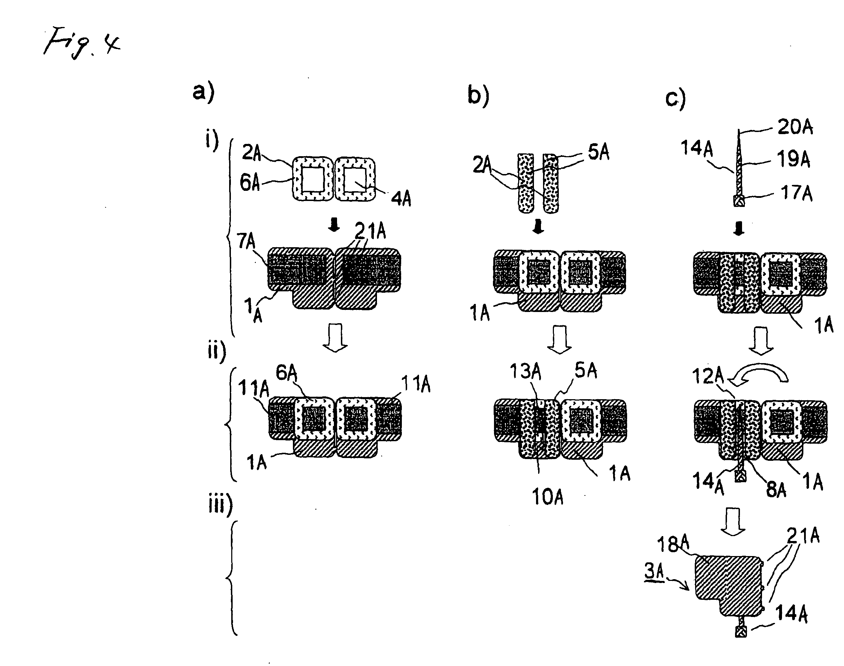

Fig. 4 shows an example of manufacture of the needle integrated biosensor according to the present invention. Figs. 4a) to c) show an examples of preparation of the needle integrated biosensor in which i) shows constituting materials for the preparation of the needle integrated biosensor and ii) and iii) show the molded products thereof. Fig. 4a) shows a resist layer 6A and plate materials of the substrates 1A, 1A where electroconductive material 7A and connecting part 21A by a broken line on the surface of the biosensor. The resist layer 6A is installed not only for playing a role as a spacer 2A but also for stipulating the electrode area and for preventing the contact of the electrode surface to a puncture needle. Therefore, a penetrating hole 4A is formed in the resist layer 6A. Here, the substrates 1A, 1A are made to be used safely by rounding their corners. Fig. 4b) shows the manner where an adhesive layer 5A is formed on a resist layer. Here, the adhesive layer 5A is also installed between the substrate 1A and the cover plate material and, therefore, it plays a role of a spacer 2A as same as the resist layer 6A does. Fig. 4b)ii) shows an electrode 10A where the area is regulated by the resist layer 6A and the adhesive layer 5A and also shows the electrode reaction part 13A thereof. Fig. 4c)i) shows the constitution of the puncture needle part 14A and there is such a device that the puncture needle part 14A is constituted from a puncture needle 20A and a support 19A which supports it as well as a connecting part 17A for the outer drive and the outer drive connecting part 17A is connected to a measuring apparatus equipped with a piercing drive whereby a piercing drive from the measuring apparatus is available. In Fig. 4c), there is noted the state that the puncture needle part 14A is aligned along the sample-conveying path 8. As shown by this drawing, the puncture needle part 14A has such a structure that its contact to the electrode surface 10A is able to be avoided by the formation of a resist layer 6A. Therefore, since the contact of the reagent layer 13A to the puncture needle part 14A is able to be prevented if the reagent layer 13A is formed on the surface of the electrode 10A, the result is that pollution of the puncture needle 20A by the reagent is able to be prevented. Fig. 4c)iii) shows a needle integrated biosensor 3A as a folded molding 18A formed by folding the two substrates 1A, 1A along the connecting part 21A. The characteristic feature of the needle integrated biosensor according to the present invention is that a biosensor (folded molding 18A) is able to be assembled by installing a connective part 21A shown as a broken line on a sheet of plane substrate. Unlike the product by a conventional manufacturing method by means of layering, the biosensor assembled by such a folding system does not need piling of the substrate with a cover whereby a manufacturing method is able to be simplified. Accordingly, that is a method which is suitable for a large-scale production of a sensor being highly precisely molded.

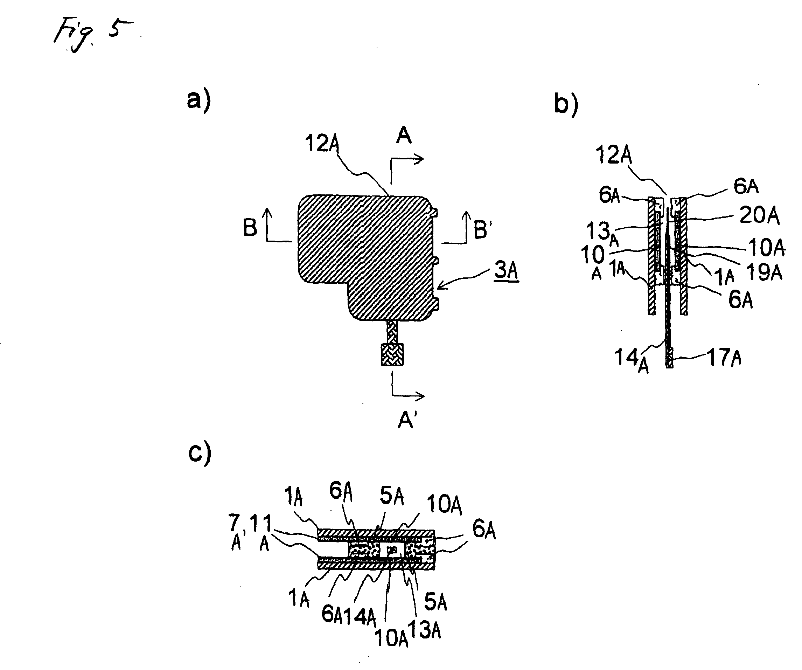

Fig. 5 shows a cross-sectional view of the needle integrated biosensor 3 shown by Fig. 4. Fig. 5b) shows a cross-sectional view along A-A' shown by Fig. 5a). As the drawing shows, a puncture needle 14A is aligned on the pattern surface installed on the substrate 1 of the biosensor. Fig. 5c) shows a cross-sectional view along B-B' shown by Fig. 5a). A puncture needle 14A is aligned at the center of the two substrates 1A, 1A. As those drawings show, the structure of the needle integrated biosensor 3A according to the present invention is in a structure of face-to-face electrodes where the electrodes 10A formed in the inner areas of the two substrates 1A, 1A are adhered in the face-to-face manner. Further, since the electrode 10A is aligned at right angles to the puncture needle 14A, the terminal 11A is able to be made outside the orbit of the puncture needle 14A. Still further, since the terminal 11A is positioned at the area which is out of the orbit of the puncture needle 14A, shape of the needle integrated biosensor 3A is bilaterally asymmetric where the puncture needle is a center line and, since that is a guide for users, no error in insertion into the measuring apparatus whether in right or left direction takes place and the measuring apparatus is also able to be equipped with a mechanism for specifying the position of the terminal 11A of the needle integrated biosensor 3A according to the present invention. Moreover, when the electrode width and distance between electrodes are made small, width of the substrate at that area also becomes small whereby it is now possible to make the amount of the sample liquid small.

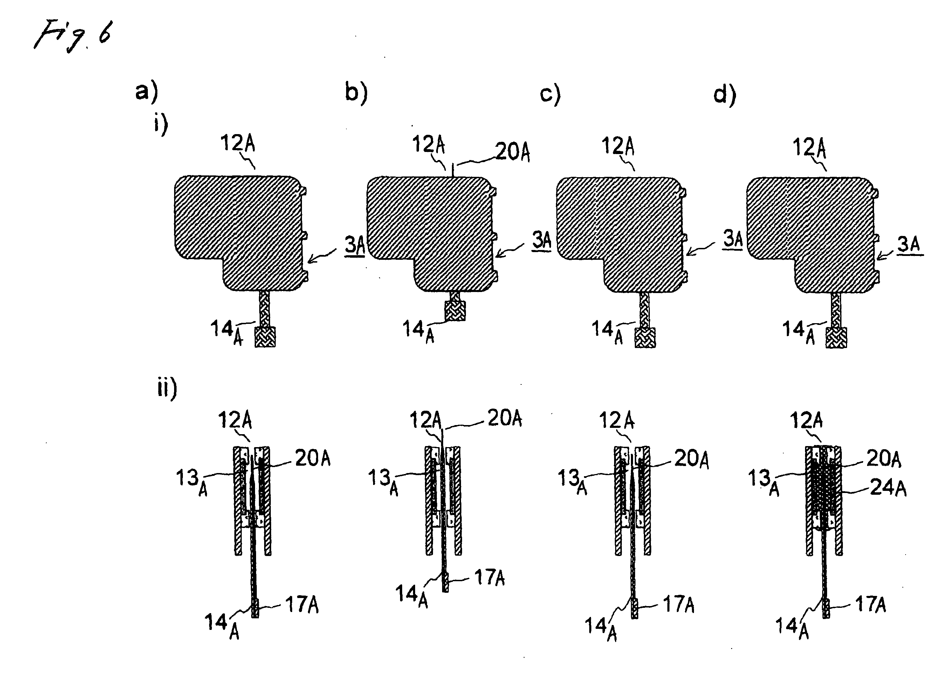

Fig. 6 shows an example of use of the needle integrated biosensor 3A shown in Fig. 5. In Fig. 6, each of a) to d) shows each of the steps where i) and ii) show the state of the needle integrated biosensor 3A at that time in which i) is in terms of a constitutional drawing and ii) is in terms of a cross-sectional view along A-A' as shown in Fig. 5a). Fig. 6a) shows the state before use of the needle integrated biosensor 3A connected to a measuring apparatus equipped with a pierce driving. At that time, the skin as a person to be tested is closely adhered to the opening 12 for piercing and collecting the blood in the needle integrated biosensor 3A. Fig. 6b) shows the piercing state and, although not shown in the drawing, the puncture needle 20A projects from the sensor to pierce the skin. Fig. 6c) shows the state when the puncture needle 14A returns to the original position after the piercing. Fig. 6d) shows the state where the blood 24A collected from the pierced skin is sucked thereafter by means of capillary phenomenon.

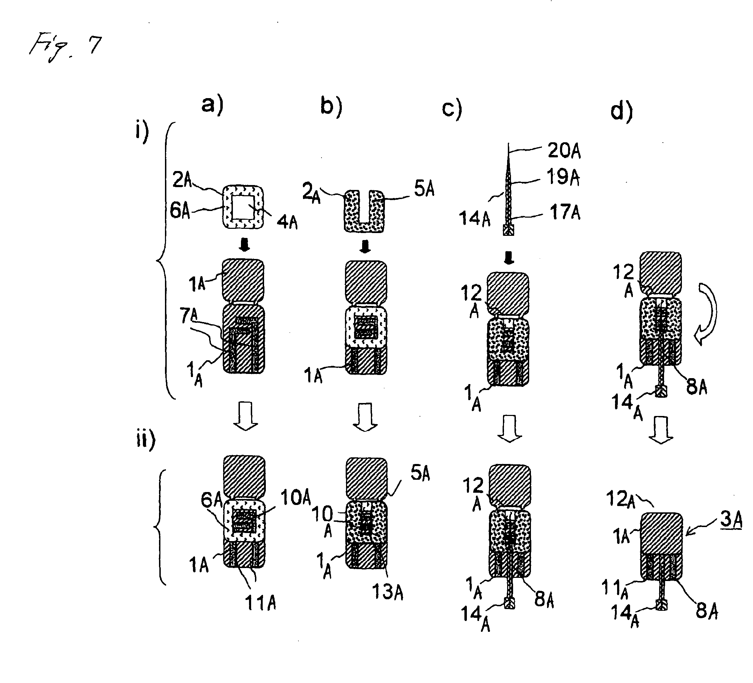

Fig. 7 shows another example of manufacture of the needle integrated biosensor according to the present invention. Figs. 7a) to d) are examples of preparation of the needle integrated biosensor in which i) shows constituting materials needed for the preparation of the folded needle integrated biosensor and ii) and iii) show the moldings thereof. Fig. 7a) shows a resist layer 6A and a plate material where an electroconductive material 7A is formed on the surface of the substrate 1A of the biosensor. Fig. 7) shows the state where an adhesive layer is formed on the resist layer. Fig. 7b)ii) shows an electrode 10A where the area is regulated by the resist layer 6A and the adhesive layer 5 and also shows the electrode reaction part 13A thereof. From Fig. 7c), the state where the puncture needle parts 14A are aligned along the sample-conveying path 8A is noted. Further, Fig. 7d) shows the needle integrated biosensor as a folded molding 18A formed by folding and piling of the two substrates 1A, 1A along the connective part 21A.

### Example 2

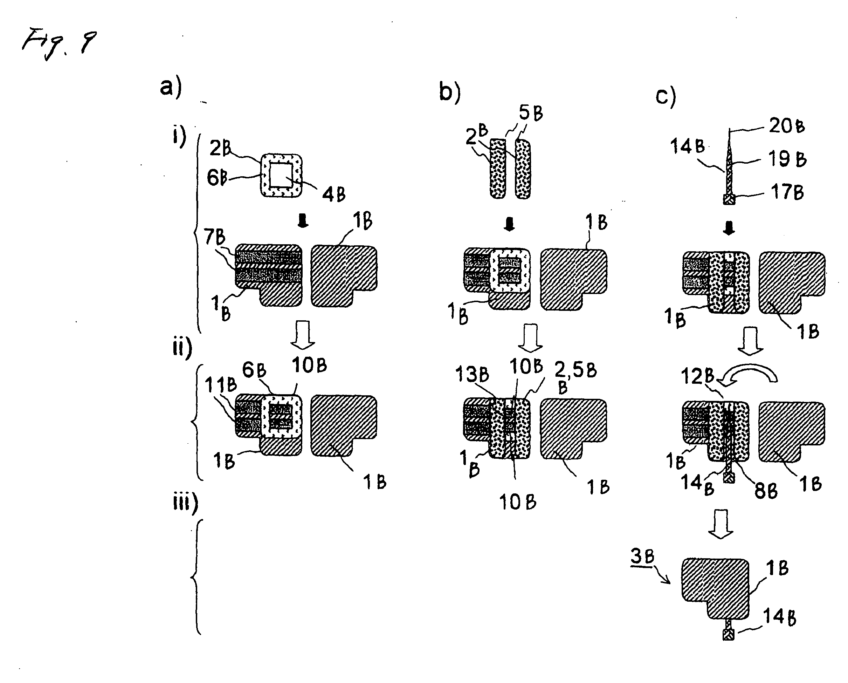

Fig. 9 shows an example of assembling the needle integrated biosensor according to the present invention. Fig. 9a) to c) are examples of manufacture of the needle integrated biosensor in which i) shows constituting materials needed for the manufacture of the needle integrated biosensor and ii) and iii) show moldings thereof. Fig. 9a) shows a resist layer 6B and a product where electroconductive materials 7B, 7B are formed on one of the surfaces of the substrates 1B, 1B of a biosensor. The resist layer 6B is installed not only for playing a role of a spacer 2B but also for regulating the area of the electrode and for preventing the contact of the electrode surface to the puncture needle. Accordingly, the resist layer 6B is equipped with a penetrated hole 4B. Here, the substrate 1B is rounded in the corners so that it is able to be used safely. Fig. 9b) shows the state where an adhesive layer 5B is formed on the resist layer. Here, since the adhesive layer 5B is also installed between the plate materials the substrates 1B, 1B, it plays a role of a spacer 2B the same as the resist layer 6B. Fig. 9b)ii) shows an electrode 10B where its area is regulated by the resist layer 6B and the adhesive layer 5B and also shows the electrode reaction part 13B thereof. Fig. 9c)i) shows the constitution of the puncture needle part 14B. The puncture needle part 14B is constituted from a puncture needle 20B, a support 19B for supporting it and a connecting part 17B to the outer drive and there is such a constitution that the outer drive connecting part 17B is connected to a measuring apparatus equipped with a piercing drive whereby a piercing drive is achieved from the measuring apparatus. From Fig. 9c), it is noted the state where the puncture needle part 14B is aligned by crossing at right angles to the electrode along the sample-conveying path 8B. As the drawing shows, the puncture needle part 14B adopts a structure where its contact to the electrode surface 10B is avoided by formation of a resist layer 6B. Accordingly, even when the reagent layer 13B is formed on the surface of the electrode 10B, contact of the reagent layer 13B to the puncture needle part 14B is able to be prevented and, therefore, pollution of the puncture needle 20B by the reagent is able to be prevented as a result thereof. Fig. 9c)iii) shows the needle integrated biosensor 3B which is formed as such.

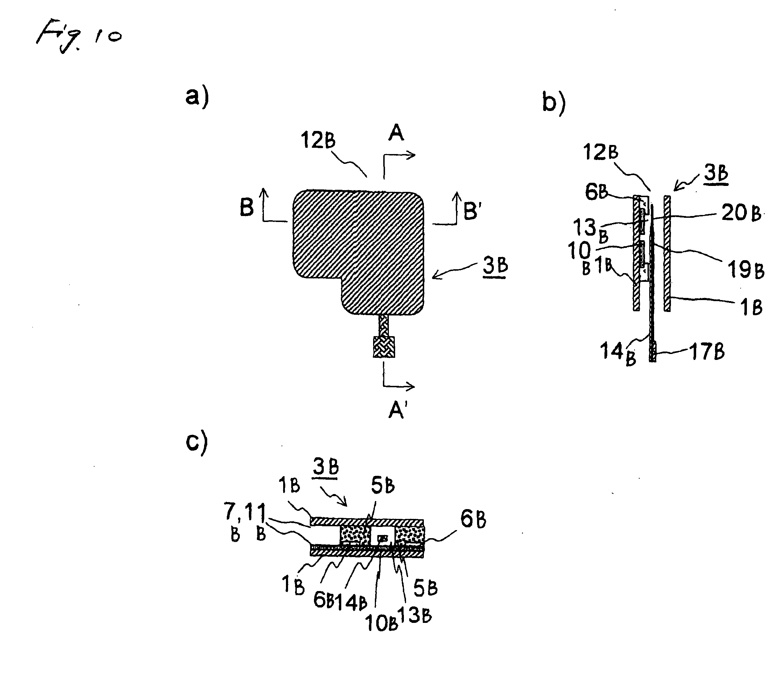

Fig. 10 shows a cross-sectional view of the needle integrated biosensor 3B shown in Fig. 9. Fig. 10b) is a cross-sectional view along A-A' shown in Fig. 10a). As the drawing shows, a puncture needle 14B is aligned on the pattern surface installed on the substrate for the biosensor. Fig. 10c) shows a cross-sectional view along B-B' shown in Fig. 10a). As those drawings show, in the needle integrated biosensor 3B according to the present invention, a puncture needle 14B is aligned in right angles to the long-axial direction of the electrode 10B, 10B formed in the inner area of one substrate 1B whereby the terminal 11B is able to be made outside of the orbit of the puncture needle 14B. Further, since the terminal 11B is aligned in the position out of the orbit of the puncture needle 14B, the shape of the needle integrated biosensor 3B is bilaterally asymmetric where the puncture needle is a center line and a user is able to adopt it as a mark so that an error for right and left in inserting into a measuring apparatus is able to be avoided and the measuring apparatus is also able to be equipped with a mechanism for specifying the position of the terminal of the needle integrated biosensor according to the present invention. Still further, when width of the electrode and distance between electrodes are made small, the width of the substrate at that area becomes small as well whereby it is able to be attempted to make the amount of sample liquid small.

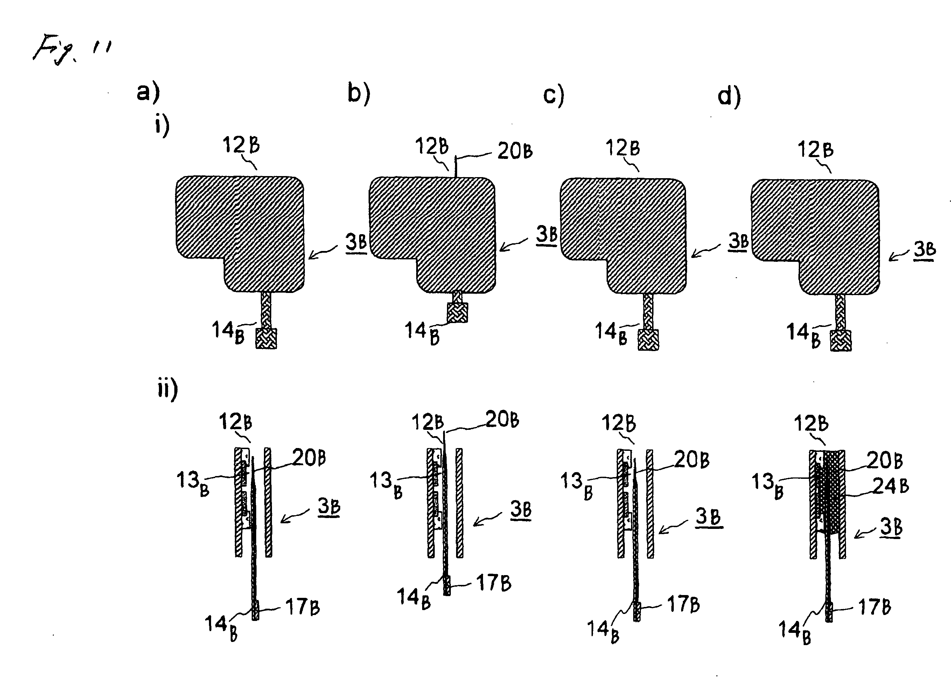

Fig. 11 shows an example of the use of the needle integrated biosensor 3B shown in Figs. 9 and 10. In Fig. 11, each step is shown in each of a) to d) while i) and ii) show the state of the needle integrated biosensor 3B at that time wherein i) is a constitutional drawing and ii) is a cross-sectional view along A-A' shown in Fig. 10a). Fig. 11a) shows the state before use of the needle integrated biosensor 3B connected to a measuring apparatus equipped with a piercing drive. At that time, the skin as a person to be tested is closely adhered to the opening 12B for piercing and collecting the blood of the needle integrated biosensor 3B. Fig. 11b) shows the state of piercing and, although not shown in the drawing, the puncture needle 20B projects from the sensor and breaks through the skin. Fig. 11c) shows the state where the puncture needle part 14B returns to the original position after the piercing. Fig. 11d) shows the state where the collected blood 24B from the pierced skin is sucked by a capillary phenomenon thereafter.

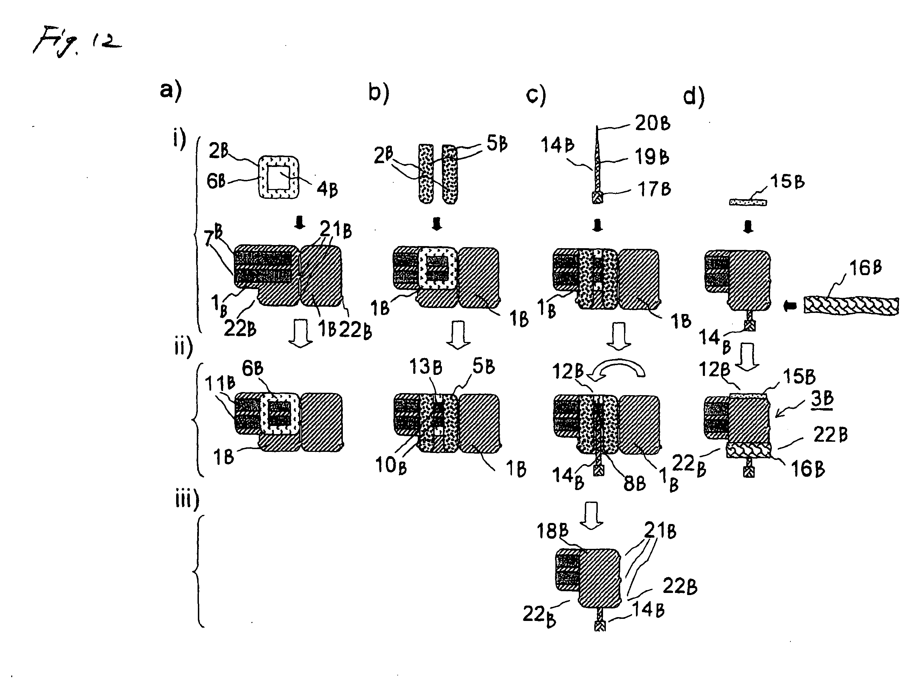

Fig. 12 shows another example of constitution of the needle integrated biosensor according to the present invention. Fig. 12a) to d) are examples for the manufacture of the needle integrated biosensor in which i) shows constituting materials for the manufacture of the needle integrated biosensor and ii) and iii) show the molded products thereof. Difference from the needle integrated biosensor shown in Fig. 9 is that a biosensor (folded assembled product 18B) is assembled by forming a connecting part such as perforation on one sheet of plane substrate and further that a sucking mechanism for collection of the blood is equipped. Firstly, the biosensor assembled by such a folded system does not need the layering of the substrates unlike the manufacturing method by a layering means in the case of Fig. 9 whereby there is a characteristic feature that the manufacturing steps are able to be simplified. Accordingly, it is able to be said to be a method which is suitable for the manufacture of a highly precisely molded sensor in a large scale with a high yield. Further, the joint which is a joint 21B of the substrates which is necessary for a folded structure also acts as a hook 22B for fixing the shrinking material 16B to the substrate 1B as shown in Fig. 12d)ii). On the other hand, this mechanism for the collection of the blood by sucking is constituted by shutting out the sample-conveying path and piercing drive part 8B from the outer air by a shrinking material 16B for tightly closing the space among the soft material 15B near to the opening 12B for piercing and collecting the blood, the substrate 1B and the puncture needle support 17B. Further, the soft material is useful for a close adhesion of the skin of the person to be tested to the opening 12B for piercing and collecting the blood.

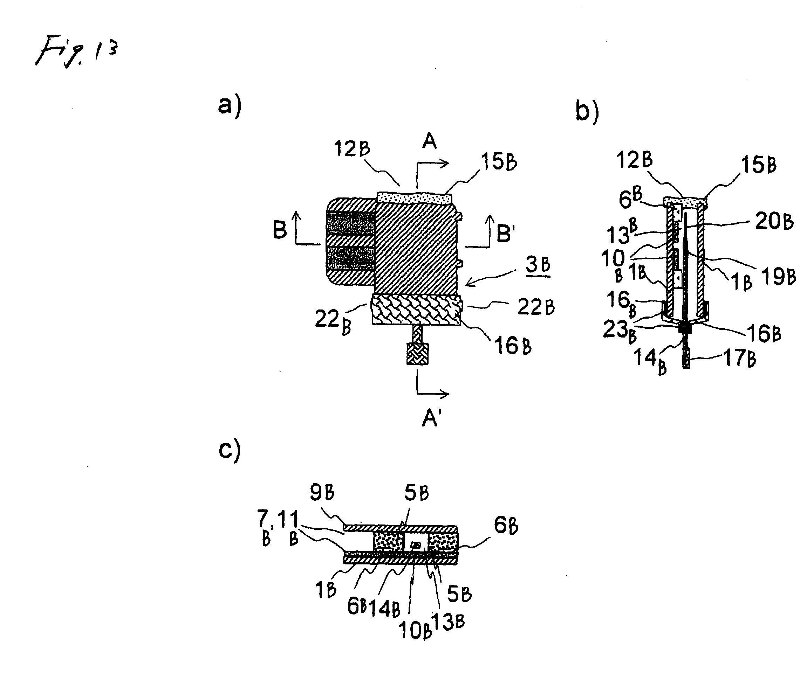

Fig. 13 shows a cross-sectional view of the needle integrated biosensor 3B shown in Fig. 12. Fig. 13b) shows a cross-sectional view along A-A' shown by Fig. 13a). As the drawing shows, a puncture needle 14B is aligned on the surface of the pattern installed on the substrate 1B of the biosensor. Further, the puncture needle part 14B is fixed by a shrinking material 16B to the substrates 1B, 1B using an adhesive part 23B. Fig. 13c) shows a cross-sectional view along B-B' shown in Fig. 13a). A puncture needle 14B is aligned at the central area of the substrates 1B, 1B. As those drawings show, the needle integrated biosensor 3B according to the present invention has such a structure that the electrode 10B is aligned by crossing at right angles to the puncture needle 14B so that the terminal 11B is able to be made outside the orbit of the puncture needle 14B whereby airtightness for keeping the inner part of the sample-conveying path 8B including the puncture needle 14B is able to be easily achieved. Further, when width of the electrode and distance between the electrodes are made small, the width of the substrate at that area becomes small as well whereby amount of the sample liquid is able to be made small. Still further, since the terminal 11B is aligned at the position far from the orbit of the puncture needle 14B, the shape of the needle integrated biosensor 3B becomes bilaterally asymmetric where the puncture needle is a center line and, therefore, a user is able to adopt it as a mark so that an error for right and left in inserting into a measuring apparatus is able to be avoided and the measuring apparatus is also able to be equipped with a mechanism for specifying the position of the terminal 11B of the needle integrated biosensor 3B according to the present invention.

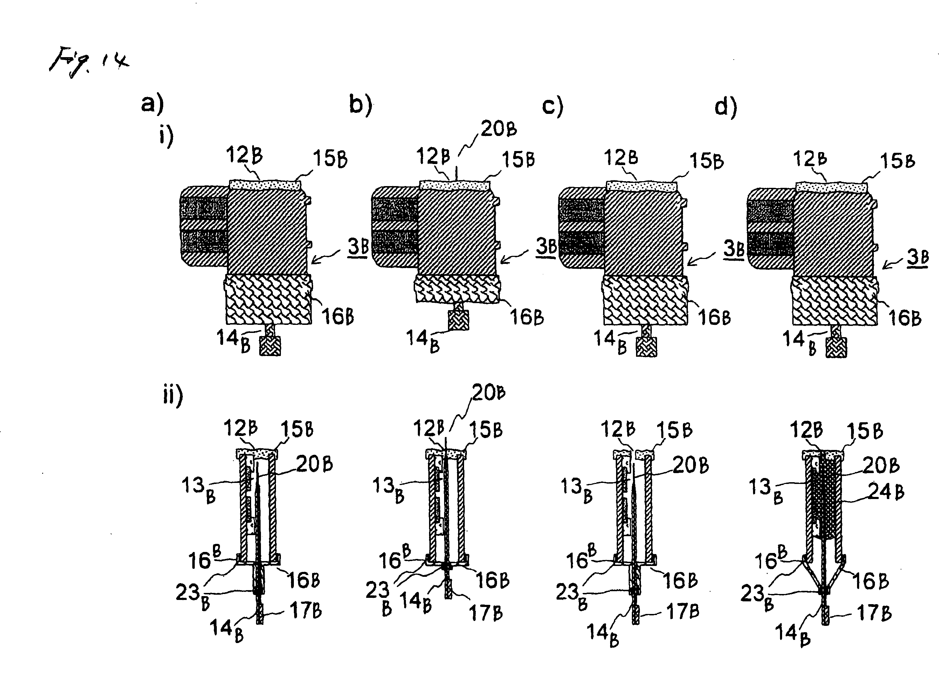

Fig. 14 shows an example of the use of the needle integrated biosensor 3B shown in Figs. 12 and 13. Fig. 14 shows each of the steps in each of a) to d) and i) and ii) show the state of the needle integrated biosensor 3B at that time in which i) shows a constitutional drawing and ii) shows a cross-sectional view along A-A' shown in Fig. 13a). Fig. 14a) shows the state of the needle integrated biosensor 3B connected to the measuring apparatus equipped with a piercing drive before use. At that time, the skin as a person to be tested is closely adhered to the soft material 15B installed at the opening 12B for piercing and collecting the blood of the needle integrated biosensor 3B. Fig. 14b) shows the state of piercing and shows the state where the puncture needle 20B penetrates through the soft material 15B. Although not shown in the drawing, the puncture needle 20B also penetrates the skin at that time. It is noted that the shrinking material 16B is shrinking. Fig. 14c) shows the state where the puncture needle part 14B returns to the original position after the piercing. Here, a soft material 15B penetrated by the puncture needle 20B is shown. In this state, negative pressure in the biosensor is applied to the pierced skin. Fig. 14d) shows that the bleeding 24B from the skin after piercing is sucked by the negative pressure of the inside area thereafter.

### Example 3

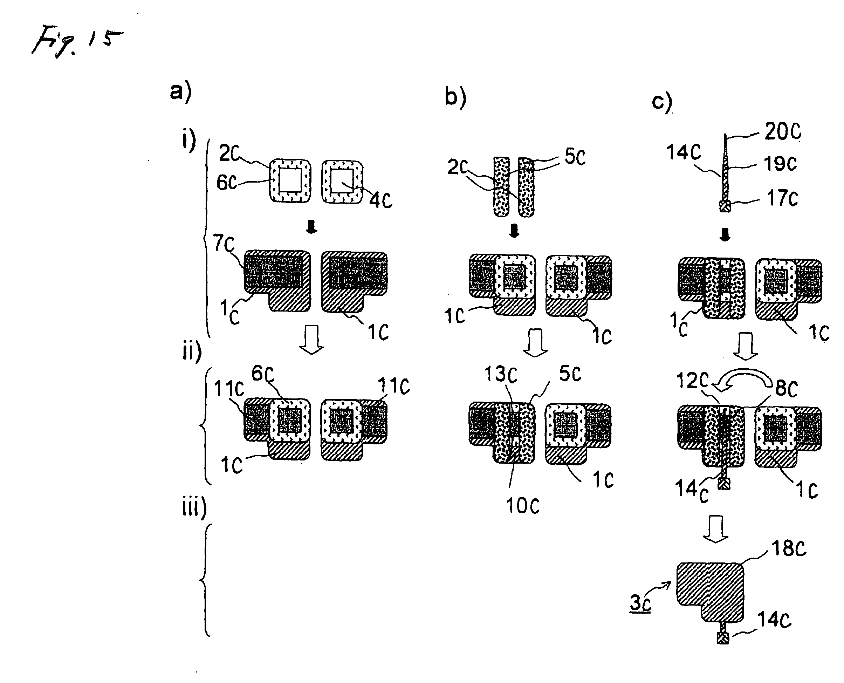

Fig. 15 shows an example of assembling the needle integrated biosensor according to the present invention. Fig. 15a) to c) are examples of manufacture of the needle integrated biosensor in which i) shows constituting materials needed for the manufacture of the needle integrated biosensor and ii) and iii) show moldings thereof. Fig. 15a) shows a resist layer 6C and a product where electroconductive material 7C is formed on the surface of each of two plate materials as the substrates 1C, 1C of a biosensor. The resist layer 6C is installed not only for playing a role of a spacer 2C but also for regulating the area of the electrode and for preventing the contact of the electrode surface to the puncture needle. Accordingly, the resist layer 6C is equipped with a penetrated hole 4C. Here, the substrate 1C is rounded in the corners so that it is able to be used safely. Fig. 15b) shows the state where an adhesive layer 5C is formed on the resist layer. Here, since the adhesive layer 5C is also installed between the plate materials of the substrates 1C, 1C, it plays a role of a spacer 2B the same as the resist layer 6C. Fig. 15b)ii) shows an electrode 10C where its area is regulated by the resist layer 6C and the adhesive layer 5C and also shows the electrode reaction part 13C thereof. Fig. 15c)i) shows the constitution of the puncture needle part 14C. The puncture needle part 14C is constituted from a puncture needle 20C, a support 19C for supporting it and a connecting part 17C to the outer drive and there is such a constitution that the outer drive connecting part 17C is connected to a measuring apparatus equipped with a piercing drive whereby a piercing drive is achieved from the measuring apparatus. From Fig. 15c), it is noted the state where the puncture needle part 14C is aligned along the sample-conveying path 8C. As the drawing shows, the puncture needle part 14C adopts a structure where its contact to the electrode surface 10C is avoided by formation of a resist layer 6C. Accordingly, even when the reagent layer 13C is formed on the surface of the electrode 10C, contact of the reagent layer 13C to the puncture needle part 14C is able to be prevented and, therefore, pollution of the puncture needle 20C by the reagent is able to be prevented as a result thereof. Fig. 15c)iii) shows the needle integrated biosensor 3C which is formed as such.

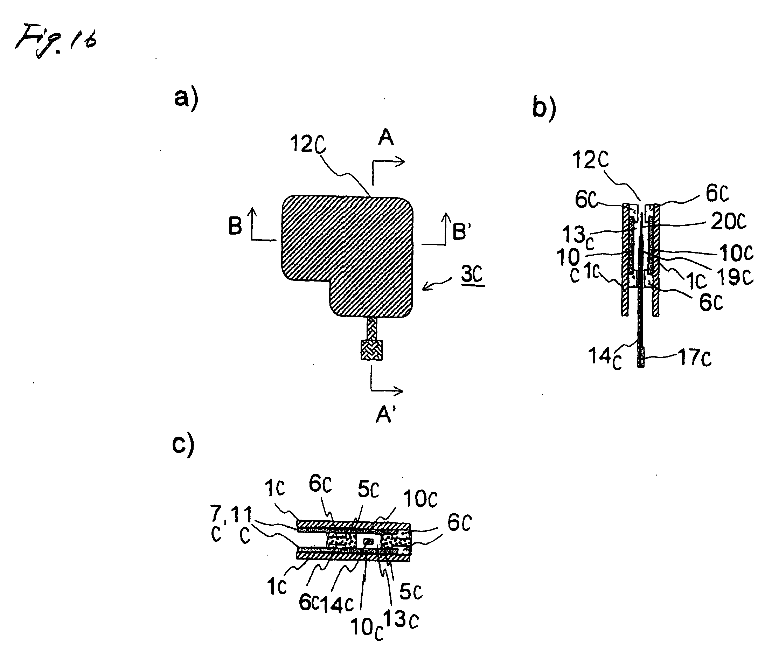

Fig. 16 shows a cross-sectional view of the needle integrated biosensor 3C shown in Fig. 15. Fig. 16b) is a cross-sectional view along A-A' shown in Fig. 16a). As the drawing shows, a puncture needle 14C is aligned on the pattern surface installed on the substrate for the biosensor. Fig. 16c) shows a cross-sectional view along B-B' shown in Fig. 16a). As those drawings show, the structure of the needle integrated biosensor 3C according to the present invention is that the electrodes 10C formed in the inner areas of the two substrates 1C, 1C are adhered in a face-to-face manner to form face-to-face electrodes. Further, the electrode 10C is aligned in a crossing manner at right angles to the puncture needle 14C whereby the terminal 11C is able to be made outside the orbit of the puncture needle 14C. Still further, since the terminal 11C is aligned in the position out of the orbit of the puncture needle 14C, the shape of the needle integrated biosensor 3C is bilaterally asymmetric where the puncture needle is a center line and a user is able to adopt it as a mark so that an error for right and left in inserting into a measuring apparatus is able to be avoided and the measuring apparatus is also able to be equipped with a mechanism for specifying the position of the terminal 11C of the needle integrated biosensor 3C according to the present invention. Furthermore, when width of the electrode and distance between electrodes are made small, the width of the substrate at that area becomes small as well whereby it is able to be attempted to make the amount of sample liquid small.

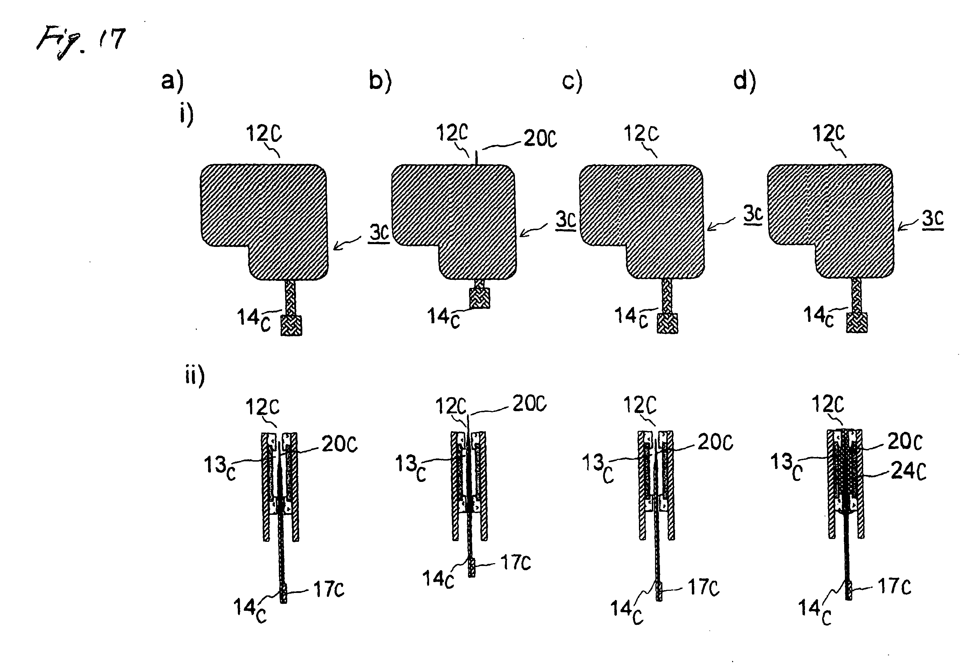

Fig. 17 shows an example of the use of the needle integrated biosensor 3C shown in Figs. 15 and 16. In Fig. 17, each step is shown in each of a) to d) while i) and ii) show the state of the needle integrated biosensor 3B at that time wherein i) is a constitutional drawing and ii) is a cross-sectional view along A-A' shown in Fig. 16a). Fig. 17a) shows the state before use of the needle integrated biosensor 3C connected to a measuring apparatus equipped with a piercing drive. At that time, the skin as a person to be tested is closely adhered to the opening 12C for piercing and collecting the blood of the needle integrated biosensor 3C. Fig. 17b) shows the state of piercing and, although not shown in the drawing, the puncture needle 20C projects from the sensor and breaks through the skin. Fig. 17c) shows the state where the puncture needle part 14C returns to the original position after the piercing. Fig. 17d) shows the state where the collected blood 24C from the pierced skin is sucked by a capillary phenomenon thereafter.

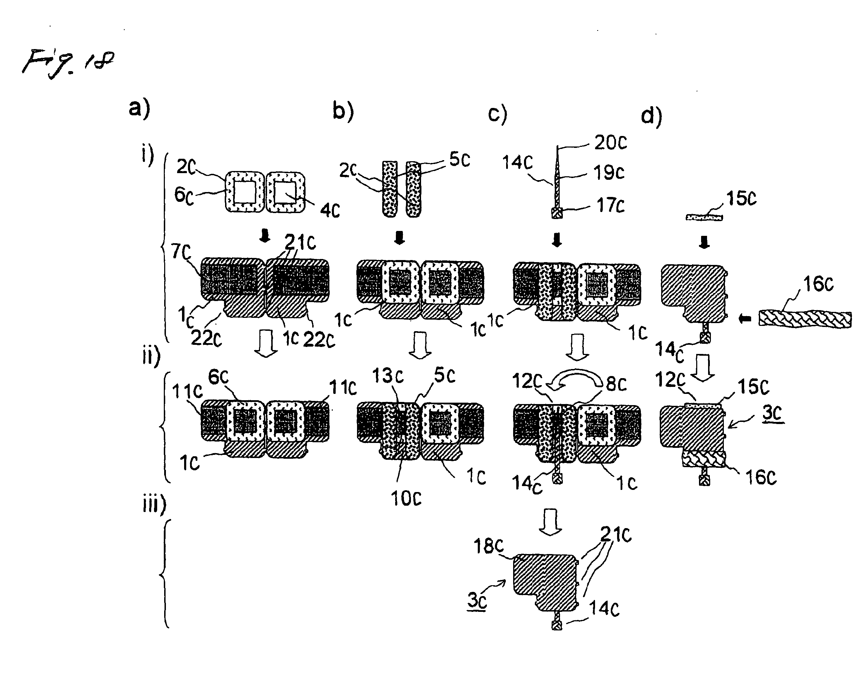

Fig. 18 shows another example of constitution of the needle integrated biosensor according to the present invention. Figs. 18a) to d) are examples for the manufacture of the needle integrated biosensor in which i) shows constituting materials for the manufacture of the needle integrated biosensor and ii) and iii) show the molded products thereof. Difference from the needle integrated biosensor shown in Fig. 15 is that a biosensor (folded assembled product 18C) is assembled by forming a connecting part such as perforation on one sheet of plane substrate and further that a sucking mechanism for collection of the blood is equipped. Firstly, the biosensor assembled by such a folded system does not need the layering of the substrates unlike the manufacturing method by a layering means in the case of Fig. 15 whereby there is a characteristic feature that the manufacturing steps are able to be simplified. Accordingly, it is able to be said to be a method which is suitable for the manufacture of a highly precisely molded sensor in a large scale with a high yield. Further, the joint which is a joint 21C of the substrates which is necessary for a folded structure also acts as a hook 22C for fixing the shrinking material 16C to the substrate 1C as shown in Fig. 18d)ii). On the other hand, this mechanism for the collection of the blood by sucking is constituted by shutting out the sample-conveying path and piercing drive part 8C from the outer air by a shrinking material 16C for tightly closing the space among the soft material 15C near to the opening 12C for piercing and collecting the blood, the substrate 1C and the puncture needle support 17C. Further, the soft material is useful for a close adhesion of the skin of the person to be tested to the opening 12C for piercing and collecting the blood.

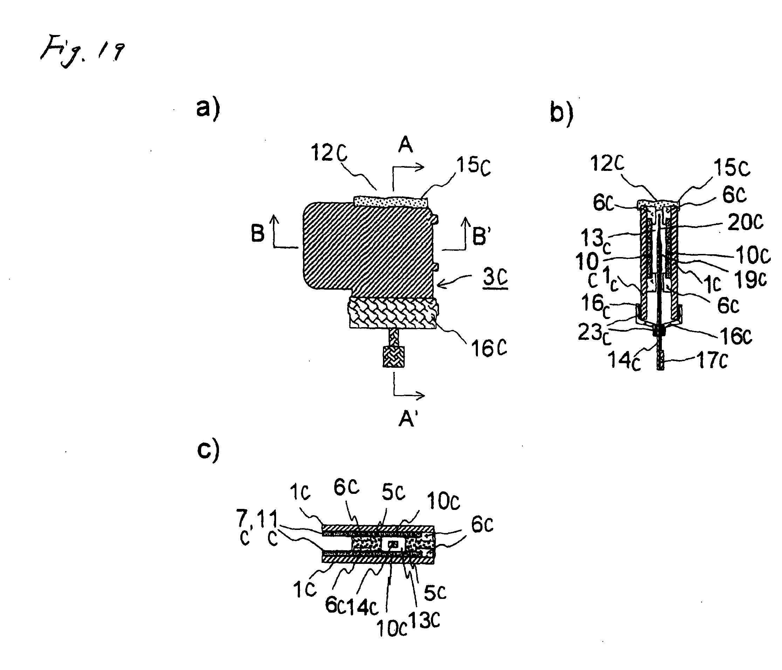

Fig. 19 shows a cross-sectional view of the needle integrated biosensor 3C shown in Fig. 18. Fig. 19b) shows a cross-sectional view along A-A' shown by Fig. 19a). As the drawing shows, a puncture needle 14C is aligned on the surface of the pattern installed on the substrate 1C of the biosensor. Further, the puncture needle part 14C is fixed by a shrinking material 16C to the substrates 1C, 1C using an adhesive part 23C. Fig. 19c) shows a cross-sectional view along B-B' shown in Fig. 19a). A puncture needle 14C is aligned at the central area of the substrates 1C, 1C. As those drawings show, the needle integrated biosensor 3C according to the present invention has such a structure that the electrode 10C is aligned by crossing at right angles to the puncture needle 14C so that the terminal 11C is able to be made outside the orbit of the puncture needle 14C whereby airtightness for keeping the inner part of the sample-conveying path 8C including the puncture needle 14C is able to be easily achieved. Further, when width of the electrode and distance between the electrodes are made small, the width of the substrate at that area becomes small as well whereby amount of the sample liquid is able to be made small. Still further, since the terminal 11C is aligned at the position far from the orbit of the puncture needle 14C, the shape of the needle integrated biosensor 3C becomes bilaterally asymmetric where the puncture needle is a center line and, therefore, a user is able to adopt it as a mark so that an error for right and left in inserting into a measuring apparatus is able to be avoided and the measuring apparatus is also able to be equipped with a mechanism for specifying the position of the terminal 11C of the needle integrated biosensor 3C according to the present invention.

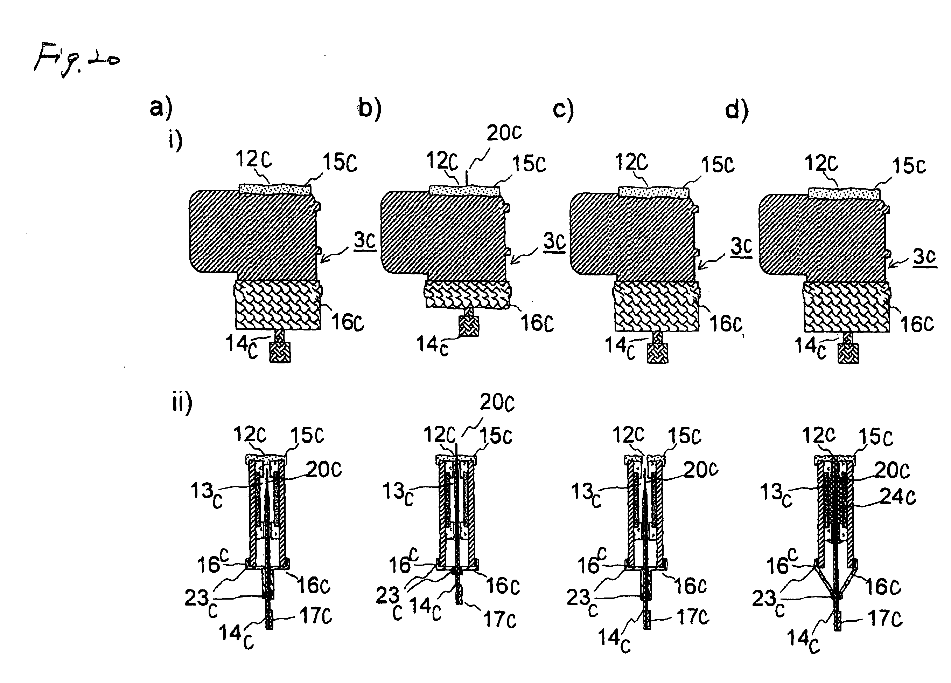

Fig. 20 shows an example of the use of the needle integrated biosensor 3C shown in Figs. 18 and 19. Fig. 20 shows each of the steps in a) to d) each and i) and ii) show the state of the needle integrated biosensor 3C at that time in which i) shows a constitutional drawing and ii) shows a cross-sectional view along A-A' shown in Fig. 19a). Fig. 20a) shows the state of the needle integrated biosensor 3C connected to the measuring apparatus equipped with a piercing drive before use. At that time, the skin as a person to be tested is closely adhered to the soft material 15C installed at the opening 12C for piercing and collecting the blood of the needle integrated biosensor 3C. Fig. 20b) shows the state of piercing and shows the state where the puncture needle 20C penetrates through the soft material 15C. Although not shown in the drawing, the puncture needle 20C also penetrates the skin at that time. It is noted that the shrinking material 16C is shrinking. Fig. 20c) shows the state where the puncture needle part 14C returns to the original position after the piercing. Here, a soft material 15C penetrated by the puncture needle 20C is shown. In this state, negative pressure in the biosensor is applied to the pierced skin. Fig. 20d) shows that the bleeding 24C from the skin after piercing is sucked by the negative pressure of the inside area thereafter.

Although the present invention is illustrated in detail by referring to specific embodiments hereinabove, it is apparent for persons skilled in the art that various changes and modifications thereof are able to be done without departing the spirit and the scope of the present invention.
The present application is on the basis of three Japanese patent applications (Application Nos. 2005-185989, 2005-185990 and 2005-185991) filed on June 27, 2005 and a Japanese patent application (Application No. 2006-035937) filed on February 14, 2006 and their contents are incorporated herein as references.

## Claims

1. A biosensor comprising:
two sheets of electrically insulating substrates connected by a connecting part, and
an electrode and a spacer formed on one of or both of the two sheets of electrically insulating substrates, wherein
the two sheets of the electrically insulating substrates are folded along the connecting part, and
the electrode and the spacer are positioned between the electrically insulating substrates.

2. The biosensor according to claim 1, wherein
the connecting part is provided by preparing broken lines or forming a hinge to the electrically insulating substrate.

3. The biosensor according to claim 1, wherein
a pole insertion hole through which a pole used for positioning the two electrically insulating substrates is provided at sites where the two electrically insulating substrates confront upon forming the biosensor.

4. The biosensor according to claim 3, wherein
a pole is penetrated through the pole insertion hole to position the two electrically insulating substrates.

5. A needle integrated biosensors with a puncture needle for penetrating a skin of a person to be tested to collect a body fluid is included together with the electrode and the spacer in the biosensor according to any one of claims 1 to 4, wherein
the electrode, the spacer and the puncture needle are positioned between the electrically insulating substrates.

6. The needle integrated biosensor according to claim 5, wherein
the puncture needle is movable in the biosensor by a drive from outside.

7. A needle integrated biosensor comprising:
a biosensor including an electrode and a spacer in a space sandwiched between two sheets of electrically insulating substrate, and
a puncture needle which is provided in the biosensor to collect a body fluid by piercing a skin of a person to be tested,
the biosensor and the puncture needle being constituted in an integrated manner via a support for the puncture needle, wherein
the puncture needle is provided in a crossing manner at a right angle to a long-axial direction of the electrode formed on one of the substrates.

8. A needle integrated biosensor comprising:
a biosensor including an electrode and a spacer in a space sandwiched between two sheets of electrically insulating substrate, and
a puncture needle which is provided in the biosensor to collect a body fluid by piercing a skin of a person to be tested,
the biosensor and the puncture needle being constituted in an integrated manner via a support for the puncture needle, wherein
the electrodes are provided in a face-to-face manner to form facing electrodes.

9. The needle integrated biosensor according to claim 8, wherein
the puncture needle is provided by crossing at a right angle to the long-axial direction of the electrode in the biosensor.

10. The needle integrated biosensor according to claim 7 or 9, wherein
the biosensor has a bilaterally asymmetric shape where the puncture needle is a center line.

11. A measuring apparatus for a needle integrated biosensor, comprising:
the needle integrated biosensor mentioned in any one of claims 1 to 10,
an introducing part for the needle integrated biosensor where the needle integrated biosensor is inserted and set,
a connector part for catching electric signal at an electrode of the needle integrated biosensor,
a measuring part for measuring electric value via the connector part,
an operation panel part for measurement,
a display part for displaying a measured value at the measuring part,
a memory part for storing the measured value,
a driving part for driving a puncture needle, and
a pierce starting button for starting the piercing by a drive trigger part and the puncture needle.

12. The needle integrated biosensor according to claim 11, further comprising:
a mechanism to recognize the bilaterally asymmetric structure of the needle integrated biosensor where the puncture needle is a center line by a projected part of the terminal for the measurement.

13. The needle integrated biosensor according to claim 11 or 12, wherein
at least one of voice guidance mechanism and a voice recognition mechanism, a control mechanism for measured data by incorporation of a radio clock, a communication mechanism of the measured data to medical organizations and a charging mechanism is operated.

14. A method for manufacturing a biosensor, comprising the steps of:
inserting a pole for a folding guide into a pole insertion hole of a biosensor,
folding the biosensor together with the folding guide,
inserting the pole into another pole insertion hole of the biosensor, and
storing the pole in a pole receiving pole of the biosensor.
